Europäisches Patentamt

European Patent Office

Office européen des brevets

(19)

(11) **EP 1 232 726 B1**

(12) **FASCICULE DE BREVET EUROPEEN**

(45) Date de publication et mention
de la délivrance du brevet:
**15.12.2004 Bulletin 2004/51**

(51) Int Cl.[7]: **A61B 6/02**

(21) Numéro de dépôt: **02290367.8**

(22) Date de dépôt: **14.02.2002**

(54) **Procédé d'estimation d'un rayonnement diffusé, notamment afin de corriger des mesures en radiographie**

Verfahren zur Abschätzung von gestreuter Strahlung, insbesondere zur Korrektur von Röntgenaufnahmen

Method for estimating scattered radiation, in particular for correcting radiographic measurements

(84) Etats contractants désignés:
**DE GB IT**

(30) Priorité: **16.02.2001 FR 0102139**

(43) Date de publication de la demande:
**21.08.2002 Bulletin 2002/34**

(73) Titulaire: **COMMISSARIAT A L'ENERGIE ATOMIQUE**
**75752 Paris Cédex 15 (FR)**

(72) Inventeurs:
• **Darboux, Michel**
**38000 Grenoble (FR)**

• **Dinten, Jean-Marc**
**69008 Lyon (FR)**

(74) Mandataire: **Ilgart, Jean-Christophe**
**c/o Brevatome,**
**3, rue du Docteur Lancereaux**
**75008 Paris (FR)**

(56) Documents cités:
**US-A- 4 549 307      US-A- 5 615 279**
**US-A- 5 666 391**

**Description**

Le sujet de cette invention est un procédé d'estimation d'un rayonnement diffusé, dont l'application principalement envisagée est la correction de radiographies.

L'utilisation d'un rayonnement à faisceau associé à un capteur bidimensionnel, très fréquente en radiographie, présente l'inconvénient de produire sur la radiographie un rayonnement diffusé important à travers l'objet examiné. En d'autres termes, chacun des détecteurs situés derrière l'objet reçoit non seulement un rayonnement primaire, provenant directement de la source par un trajet rectiligne et ayant traversé une région bien définie de l'objet, mais un rayonnement diffusé de provenance indéterminée qui affecte la mesure et qu'il serait donc souhaitable de corriger.

Plusieurs procédés sont déjà pratiqués. C'est ainsi que le rayonnement primaire peut être mesuré seul si une collimation stricte des détecteurs et de la source est faite afin d'intercepter le rayonnement diffusé, mais ce procédé nécessite en pratique un balayage du faisceau qui est lent à accomplir, et pendant lequel on doit s'accommoder de mouvements du patient si on examine des êtres vivants.

On a aussi eu l'idée contraire de ne mesurer que le rayonnement diffusé. On dispose pour cela un réseau discontinu d'absorbeurs, comme des billes de plomb, entre l'objet et les détecteurs, pour arrêter localement le rayonnement primaire, de sorte que les détecteurs situés derrière ces absorbeurs ne mesurent que le rayonnement diffusé. Ce procédé appelé « beam stop » donne donc des tables ou nappes bidimensionnelles de valeur de rayonnement diffusé, qu'on complète par interpolation entre les détecteurs placés derrière les absorbeurs. Le rayonnement diffusé ainsi estimé est soustrait du rayonnement total mesuré séparément. Ce procédé est précis mais a l'inconvénient qu'il impose deux irradiations de l'objet et donc un doublement de la dose de rayons qu'il reçoit. Un dernier exemple de méthode de correction du rayonnement diffusé par des moyens matériels comporte l'emploi de grilles anti-diffusantes, mais leur efficacité n'est que partielle ; elle est insuffisante pour un faisceau conique, où le rayonnement diffusé peut être plusieurs fois supérieur au rayonnement primaire.

Enfin, il existe un certain nombre de méthodes numériques pour estimer le rayonnement diffusé, à partir de convolutions ou de déconvolutions des mesures par exemple ; on pourrait aussi citer le brevet français 2 759 800 pour un procédé numérique différent, analytique. Elles sont en général d'emploi délicat car elles dépendent de paramètres choisis par l'utilisateur (noyaux de convolution par exemple) qui ne donnent de bons résultats que dans des situations favorables, comme des petites zones où le rayonnement diffusé est faible, ou des objets au contenu relativement homogène. Il n'existe aucun procédé simple qui permette par exemple de corriger le rayonnement diffusé à travers le thorax ou d'autres grandes zones anatomiques, dont l'examen est fréquent mais qui sont défavorables pour corriger le rayonnement diffusé en raison de leur volume même et de l'hétérogénéité due à la présence d'une structure d'os complexe et dont la capacité d'atténuation du rayonnement est très différente de celle des tissus mous.

Mentionnons enfin le brevet américain 6 018 565 pour l'exposé d'une méthode mixte, à « beam stop » et convolution.

Un objet essentiel de l'invention est de proposer un procédé d'estimation et de correction de rayonnement diffusé qui puisse convenir pour des situations difficiles de radiographie.

Le procédé conforme à l'invention est, sous sa forme la plus générale, un procédé d'estimation d'un rayonnement diffusé provenant d'un rayonnement initial ayant traversé un objet en subissant une atténuation laissant passer un rayonnement total de mesure, caractérisé par :

- une prise d'une table de mesures d'un rayonnement diffusé, obtenue en faisant passer le rayonnement initial par un simulacre de l'objet,
- un calcul de coefficients de transposition entre le simulacre et l'objet, d'après le rayonnement initial, le rayonnement total de mesure à travers l'objet et un rayonnement total de mesure à travers le simulacre,
- et une pondération de la table de mesures avec les coefficients de transposition.

Avantageusement, le simulacre sera un bloc d'épaisseur constante et en une matière homogène, ayant une atténuation semblable à une matière de base de l'objet ; en général la prise de table de mesure sera une sélection dans une série de tables de mesures de rayonnement diffusé, obtenues auparavant en faisant successivement passer le rayonnement initial à travers une série respective de simulacres de l'objet, d'épaisseurs différentes mais constante ; et la sélection sera faite par comparaison d'une valeur du rayonnement total de mesure à travers l'objet et d'une valeur du rayonnement total de mesure à travers les simulacres.

Les coefficients de pondération sont généralement des rapports de valeurs d'une même fonctionnelle calculée pour l'objet et pour le simulacre. La fonctionnelle utilisée peut être égale au produit du rayonnement total de mesure par le logarithme du rapport de rayonnement total de mesure et du rayonnement initial.

L'invention sera maintenant décrite en référence aux figures, parmi lesquelles :

- la figure 1 est une vue générale d'une acquisition des mesures ;
- la figure 2 est une vue d'une acquisition de calibration ;

- et la figure 3 illustre les étapes du procédé.

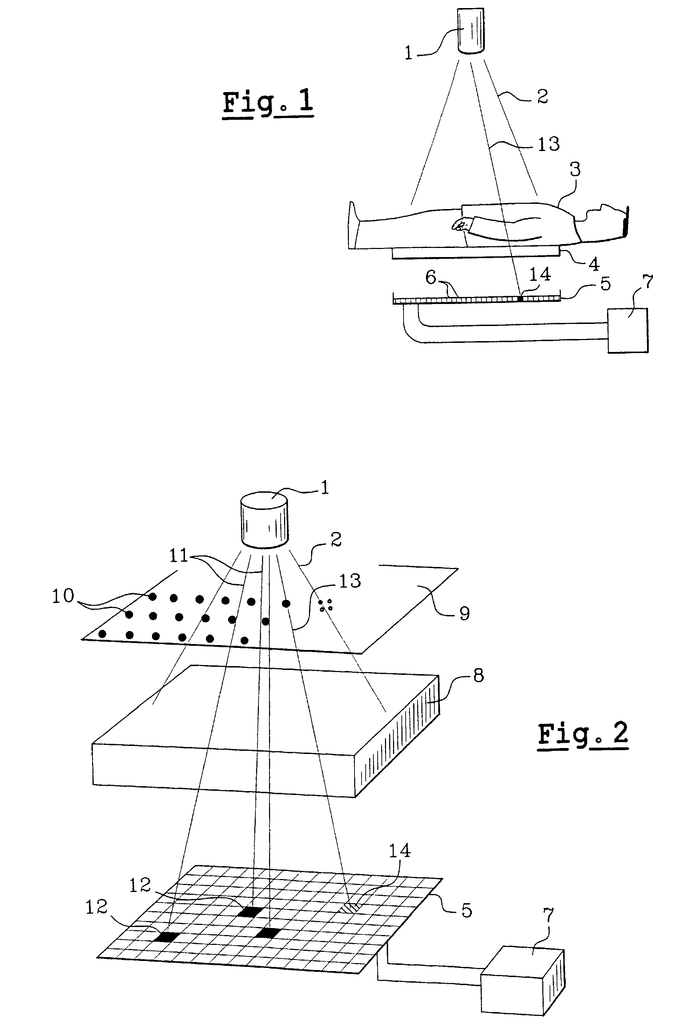

Reportons-nous d'abord à la figure 1, où un tube 1 de rayons X émet un faisceau 2 conique vers un objet 3 à examiner (ici un patient étendu sur une table 4) puis, à travers lui, vers un réseau 5 plan de détecteurs 6 disposés en matrice. Les détecteurs 6 sont reliés à un appareil d'acquisition 7 et mesurent un rayonnement diffusé qui se superpose au rayonnement primaire, seul convenable pour la radiographie.

L'estimation du rayonnement diffusé à travers le patient 3 consiste tout d'abord à obtenir des tables bidimensionnelles ou nappes de rayonnement diffusé obtenues dans des circonstances comparables. Pour cela, on effectue des irradiations d'étalonnage à travers des simulacres 8 de l'objet 30 à examiner, conformément à la figure 2 : les conditions d'irradiation restent les mêmes, c'est-à-dire qu'on continue d'utiliser le tube 1, le faisceau 2, le réseau 5 de détecteurs 6 et l'appareil d'acquisition 7, le simulacre 8 remplaçant cependant le patient ; on a aussi ajouté une grille 9 de billes 10 de plomb au dessus du simulacre 8 et du réseau 5. Il résulte de cette disposition que les rayons 11 passant par les billes 10 sont complètement absorbés et que les régions 12 du réseau 5 situées dans le prolongement de ces rayons 11 ont des détecteurs 6 qui ne mesurent que le rayonnement diffusé à ces endroits. Il suffit de relever ces valeurs mesurées et d'interpoler entre les régions 12 pour estimer convenablement le rayonnement diffusé issu du simulacre 8 pour tous les détecteurs 6 du réseau 5.

Le simulacre 8 devrait être semblable à l'objet afin que les rayonnements diffusés par eux fussent identiques. Une similitude parfaite n'est pas réalisable, et c'est pourquoi on se contente d'un simulacre 8 ressemblant à l'objet 3 et dont la nappe associée de rayonnement diffusé sera corrigée ultérieurement pour évaluer celle de l'objet. En pratique, le simulacre 8 peut être un bloc d'une matière homogène et qui présente le même coefficient d'atténuation que la matière de base de l'objet 3 : dans le cas d'un corps humain, composé pour l'essentiel de tissu mou, on sait que le plexiglas (polyméthacrylate) convient.

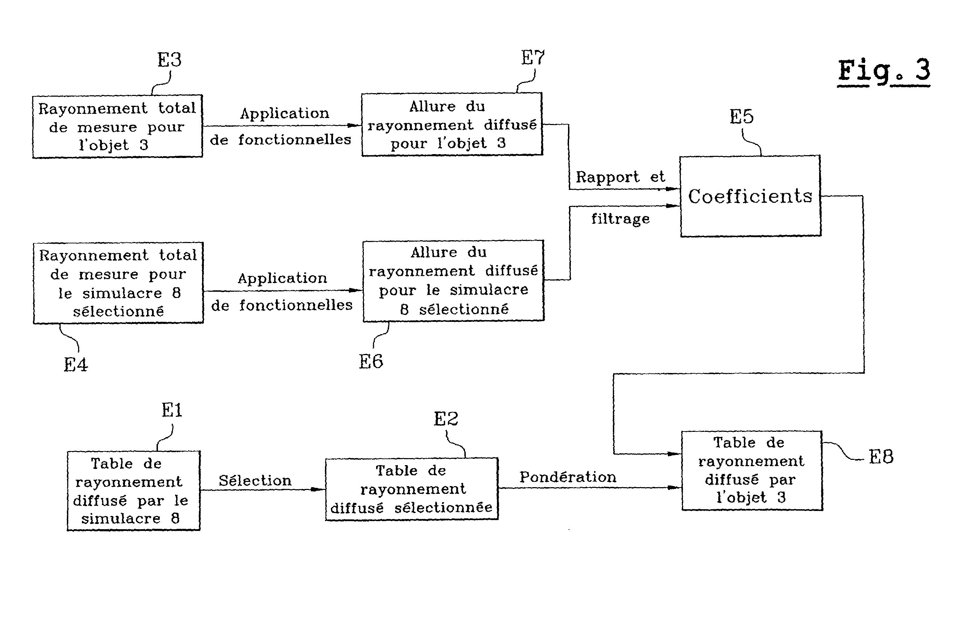

Afin de permettre des mesures variées, on disposera en réalité de plusieurs nappes de rayonnement diffusé, obtenues pour autant de simulacres 8, qui ne différeront que par leur épaisseur et donc par la longueur du trajet parcouru par les rayons 11. Ces nappes seront enregistrées dans une base de données préalablement aux mesures utiles sur les objets 3 radiographiés. pour prendre une nappe de rayonnement diffusé comparable à celle d'un objet 3, on sélectionnera en pratique une des nappes de la base de données ou, mieux, une nappe qu'on aura obtenue par des calculs d'interpolation entre deux de ces nappes. Le critère de sélection pourra être défini au moyen d'un rayon particulier 13 aboutissant à une région 14 du réseau 5 et qui ne passera ni par les absorbeurs 10 de la figure 2, ni par des tissus osseux du patient (ou plus généralement des portions de l'objet 3 dont les propriétés d'absorption sont différentes du matériau du simulacre 8) à la figure 1. Le rayonnement total, primaire et diffusé, reçu par la région 14 après avoir traversé chaque simulacre 8 servira d'index à la table de rayonnement diffusé correspondante, et la table sélectionnée aura l'index à une valeur identique au rayonnement total mesuré a la région 14 à travers l'objet 3. Tout cela correspond au passage de l'état E1 à l'état E2 dans l'organigramme de la figure 3, qu'on commence à commenter.

La suite du procédé consiste essentiellement en la correction de la table du rayonnement diffusé ainsi sélectionné pour l'ajuster au mieux qu'on puisse espérer à la nappe de rayonnement réellement diffusé par l'objet 3. Pour cela, on se sert de toutes les informations disponibles, c'est-à-dire du rayonnement total reçu par les détecteurs 6 au-delà de l'objet 3 comme du simulacre 8 sélectionné. Ce rayonnement total étant noté $\Phi t$, le rayonnement diffusé $\Phi d$, le rayonnement initial issu du tube 1 $\Phi o$ et le rayonnement primaire $\Phi$, la relation $\Phi t = \Phi + \Phi d$ est respectée.

On est alors aux états E3 et E4 de l'organigramme de la figure 3. Ensuite, on transforme les valeurs des rayonnements totaux $\Phi t$ mesurées pour l'objet 3 et le simulacre 8 sélectionné en leur appliquant des fonctionnelles. Plus précisément, il est connu dans l'art que $\Phi d$ est proportionnel au premier ordre à $\Phi \log(\Phi/\Phi o)$ ; cette relation, qui est déduite de la loi de Klein et Nishina, donne une allure générale du rayonnement diffusé, à défaut de son intensité.

Le rayonnement initial $\Phi 0$ est connu ; le rayonnement primaire $\Phi$ ne l'est pas, mais on consent à appliquer cette relation de façon approchée en le remplaçant par le rayonnement total $\Phi t$, c'est-à-dire que la fonctionnelle employée associe à chaque valeur mesurée du rayonnement total $\Phi t$ la valeur calculée $\Phi t \log(\Phi t/\Phi o)$, supposée proche du rayonnement diffusé $\Phi d$ à cet endroit ; on est parvenu aux états E5 et E6 de l'organigramme.

L'étape suivante consiste à faire, pour chacun des détecteurs 6, le rapport des valeurs données par la fonctionnelle pour l'objet 3 et le simulacre 8 sélectionné selon la formule $K = \dfrac{\Phi t \log(\Phi t/\Phi o) \text{ objet}}{\Phi t \log(\Phi t/\Phi o) \text{ simulacre}}$. Les coefficients de pondération K ainsi obtenus serviront à déformer la nappe de rayonnement diffusé sélectionnée à l'état E2 afin d'estimer celle de l'objet 3. Les résultats constituent encore une table bidimensionnelle ou une matrice ayant des dimensions identiques à celle des tables de rayonnement puisqu'elle est associée au réseau 5 de détecteurs 6. Il est donc possible et avantageux d'effectuer un filtrage numérique spatial de cette matrice en appliquant un filtre passe-bas qui corrige les coefficients K en ne conservant que les fréquences les plus basses de leur variation et de les rendre ainsi probablement plus conformes à la réalité puisque le rayonnement diffusé varie assez lentement d'un point à un autre.

Quand la table des coefficients de pondération définitifs, notés K', a été obtenue (à l'état E7), elle sert à pondérer la table de rayonnement diffusé sélectionnée auparavant à l'état E2, pour obtenir une table de rayonnement diffusé par l'objet 3 (état E8, qui constitue l'estimation recherchée) ; la formule appliquée est $\Phi d$ objet=K' $\Phi d$ simulacre. Ces valeurs estimées $\Phi d$ objet pourront alors être soustraites du rayonnement total $\Phi t$ mesuré par les détecteurs 6 pour estimer le rayonnement primaire $\Phi$ et obtenir une image radiographique plus précise de l'objet 3.

Ce procédé s'applique aux radiographies à énergie d'irradiation simple ou multiple ; dans le second cas, il est répété séparément pour chacune des énergies employées.

La fonctionnelle proposée ici n'est pas la seule qu'on puisse employer, et la fonctionnelle plus simple $\Phi d=k\Phi$ (approchée ici encore en $\Phi d=k\Phi t$), k étant une constante, pourrait aussi donner de bons résultats pour estimer $\Phi d$.

## Revendications

1. Procédé d'estimation d'un rayonnement diffusé provenant d'un rayonnement initial ayant traversé un objet (3) en subissant une atténuation laissant passer un rayonnement total de mesure, **caractérisé par** :

   - une prise d'une table de mesures d'un rayonnement diffusé, obtenue en faisant passer le rayonnement initial par un simulacre (8) de l'objet,
   - un calcul de coefficients (K') de transposition entre le simulacre et l'objet, d'après le rayonnement initial ($\Phi o$), le rayonnement total de mesure à travers l'objet ($\Phi t$ objet) et un rayonnement total de mesure à travers le simulacre ($\Phi t$ simulacre),
   - et une pondération de la table de mesures avec les coefficients de transposition.

2. Procédé d'estimation d'un rayonnement diffusé selon la revendication 1, **caractérisé en ce que** le simulacre (8) est un bloc d'épaisseur constante et en une matière homogène, ayant une atténuation semblable à une matière de base de l'objet.

3. Procédé d'estimation d'un rayonnement diffusé selon la revendication 1, **caractérisé en ce que** la prise de table de mesures est une sélection dans une série de tables de mesures de rayonnement diffusé, obtenues en faisant successivement passer le rayonnement initial à travers une série respective de simulacres de l'objet, qui sont des blocs d'épaisseurs différentes mais constante et en une matière homogène, ayant une atténuation semblable à une matière de base de l'objet.

4. Procédé d'estimation d'un rayonnement diffusé selon la revendication 3, **caractérisé en ce que** la sélection comprend une interpolation entre deux des tables de mesures.

5. Procédé d'estimation d'un rayonnement diffusé selon l'une quelconque des revendications 3 et 4, **caractérisé en ce que** la sélection est faite par comparaison d'une valeur du rayonnement total de mesure à travers l'objet et d'une valeur du rayonnement total de mesure à travers les simulacres.

6. Procédé d'estimation d'un rayonnement diffusé selon la revendication 5, **caractérisé en ce que** la comparaison est faite pour des rayons identiques (13) du rayonnement initial à travers l'objet et les simulacres, ne traversant que la matière de base de l'objet.

7. Procédé d'estimation d'un rayonnement diffusé selon l'une quelconque des revendications 1 à 6, **caractérisé en ce que** les coefficients de pondération sont des rapports de fonctionnelle identiques calculées pour l'objet et pour le simulacre.

8. Procédé d'estimation d'un rayonnement diffusé selon la revendication 7, **caractérisé en ce que** les fonctionnelles sont égales au produit du rayonnement total de mesure par le logarithme du rapport du rayonnement total de mesure et du rayonnement initial.

9. Procédé d'estimation d'un rayonnement diffusé selon l'une quelconque des revendications 1 à 8, **caractérisé en ce qu'**il comprend une étape de filtrage passe-bas des coefficients de transposition, arrangés en une table superposable à la table de mesures.

10. Procédé de radiographie comprenant une étape de correction de mesures de radiographie par une soustraction d'un rayonnement diffusé estimé selon le procédé selon l'une quelconque des revendications précédentes.

## Claims

1. Method for estimation of scattered radiation coming from an initial radiation having crossed a subject (3) and undergoing attenuation letting pass a total measurement radiation **characterised by**:

   - drawing up a table of measurements for scattered radiation, obtained by passing the initial radiation through a simulacrum (8) of the subject,
   - calculating coefficients (K') for transposition between the simulacrum and the subject, according to the initial radiation ($\Phi$o), the total measurement radiation through the object ($\Phi$t subject) and a total measurement radiation through the simulacrum ($\Phi$t simulacrum),
- and weighting the measurements table with the transposition coefficients.

2. Method for estimation of scattered radiation according to claim 1, **characterised in that** the simulacrum (8) is a block of constant thickness and of a homogeneous material, having an attenuation similar to the base material of the subject.

3. Method for estimation of scattered radiation according to claim 1, **characterised in that** drawing up the measurements table involves a selection in a series of tables of measurements for scattered radiation, obtained by making the initial radiation pass successively through a respective series of simulacra of the subject, which are blocks with different but constant thicknesses and made of a homogeneous material, with attenuation similar to a base material of the subject.

4. Method for estimation of scattered radiation according to claim 3, **characterised in that** the selection comprises an interpolation between two of the measurement tables.

5. Method for estimation of scattered radiation according to one or the other of claims 3 and 4, **characterised in that** the selection is made by comparison of a value for the total measurement radiation through the subject and a value for the total measurement radiation through the simulacra.

6. Method for estimation of scattered radiation according to claim 5, **characterised in that** the comparison is carried out for identical rays (13) of the initial radiation through the subject and the simulacra, only crossing the base material of the subject.

7. Method for estimation of scattered radiation according to any one of claims 1 to 6, **characterised in that** the weighting coefficients are identical functional ratios calculated for the subject and for the simulacrum.

8. Method for estimation of scattered radiation according to claim 7, **characterised in that** the functionals are equal to the product of the total measurement radiation by the logarithm of the ratio of the total measurement radiation and the initial radiation.

9. Method for estimation of scattered radiation according to any one of claims 1 to 8, **characterised in that** it comprises a lowpass filtering stage for the transposition coefficients set out in a table able to be superposed on the measurements table.

10. Radiography method comprising a correction stage for radiography measurements by subtraction of scattered radiation estimated according to the method according to any one of the above claims.

**Patentansprüche**

1. Verfahren zur Abschätzung einer Streustrahlung, die abstammt von einer Anfangsstrahlung, welche ein sie abschwächendes Objekt (3) durchquert, das eine Gesamtmessstrahlung passieren lässt, **gekennzeichnet durch**:

   - die Realisierung einer Messtabelle einer Streustrahlung, die man erhält, indem man die Anfangsstrahlung eine Simulation (8) des Objekts durchqueren lässt,
   - eine Berechnung von Transpositionskoeffizienten (K') zwischen der Simulation und dem Objekt gemäß der Anfangsstrahlung ($\Phi_O$), der Gesamtmessstrahlung **durch** das Objekt hindurch ($\Phi_{t\ Objekt}$) und einer Gesamtmessstrahlung **durch** die Simulation hindurch ($\Phi_{t\ Simulation}$),
   - und eine Gewichtung der Messtabelle mit den Transpositionskoeffizienten.

2. Verfahren zur Abschätzung einer Streustrahlung nach Anspruch 1, **dadurch gekennzeichnet, dass** die Simulation (8) ein Block von konstanter Dicke und aus einem homogenen Stoff ist, der eine ähnliche Schwächung wie ein Basisstoff des Objekts bewirkt.

3. Verfahren zur Abschätzung einer Streustrahlung nach Anspruch 1, **dadurch gekennzeichnet, dass** die Realisierung der Messtabelle eine Selektion aus einer Serie von Streustrahlungs-Messtabellen ist, die man erhält, indem man nacheinander die Anfangsstrahlung eine Serie von Simulationen des Objekts durchqueren lässt, die jeweils Blöcke von unterschiedlicher aber konstanter Dicke und aus einem homogenen Stoff sind, der eine ähnliche Schwächung wie ein Basisstoff des Objekts bewirkt.

4. Verfahren zur Abschätzung einer Streustrahlung nach Anspruch 3, **dadurch gekennzeichnet, dass** die Selektion eine Interpolation zwischen zwei Messtabellen umfasst.

5. Verfahren zur Abschätzung einer Streustrahlung nach einem der Ansprüche 3 und 4, **dadurch gekennzeichnet, dass** die Selektion durch Vergleich eines Messwerts der gesamten das Objekt durchquerenden Strahlung und eines Messwerts der gesamten die Simulationen durchquerenden Strahlung erfolgt.

6. Verfahren zur Abschätzung einer Streustrahlung nach Anspruch 5, **dadurch gekennzeichnet, dass** der Vergleich für identische, das Objekt und die Simulationen durchquerende Strahlen (13) der Anfangsstrahlung durchgeführt wird, die nur den Basisstoff des Objekts durchqueren.

7. Verfahren zur Abschätzung einer Streustrahlung nach einem der Ansprüche 1 bis 6, **dadurch gekennzeichnet, dass** die Gewichtungskoeffizienten identische, für das Objekt und für die Simulation berechnete Funktionalenverhältnisse sind.

8. Verfahren zur Abschätzung einer Streustrahlung nach Anspruch 7, **dadurch gekennzeichnet, dass** die Funktionalen gleich dem Produkt von Gesamtmessstrahlung mal Logarithmus des Verhältnisses von Gesamtmessstrahlung zu Anfangsstrahlung sind.

9. Verfahren zur Abschätzung einer Streustrahlung nach einem der Ansprüche 1 bis 8, **dadurch gekennzeichnet, dass** es einen Tiefpass-Filterschritt der Transpositionskoeffizienten umfasst, die in einer Tabelle angeordnet sind, die man in die Messtabelle einblenden kann.

10. Radiographieverfahren, einen Schritt zur Korrektur von Radiographiemessungen durch eine Subtraktion einer Streustrahlung umfassend, die abgeschätzt wird gemäß dem Verfahren nach einem der vorangehenden Schritte.

Fig. 1

Fig. 2

Fig. 3

E3

| Rayonnement total de mesure pour l'objet 3 |

Application de fonctionnelles →

E7

| Allure du rayonnement diffusé pour l'objet 3 |

Rapport et

E5

| Coefficients |

E4

| Rayonnement total de mesure pour le simulacre 8 sélectionné |

Application de fonctionnelles →

| Allure du rayonnement diffusé pour le simulacre 8 sélectionné |

E6

filtrage

E1

| Table de rayonnement diffusé par le simulacre 8 |

Sélection →

E2

| Table de rayonnement diffusé sélectionnée |

Pondération →

| Table de rayonnement diffusé par l'objet 3 |

E8

EP 1 232 726 B1